# EUROPÄISCHE PATENTSCHRIFT

(11) **EP 0 806 934 B1**
(45) Veröffentlichungstag und Bekanntmachung des Hinweises auf die Patenterteilung: **18.07.2001**
(21) Anmeldenummer: 96900568.5
(22) Anmeldetag: 08.01.1996
(51) Int. Cl.: A61K 7/00

(54) **2-PHASEN-HAARBEHANDLUNGSMITTEL**
2-PHASE HAIR TREATMENT MEDIUM
AGENT DE SOINS CAPILLAIRES A DEUX PHASES

(30) Priorität: 17.01.1995 DE 19501184
(43) Veröffentlichungstag der Anmeldung: 19.11.1997
(73) Patentinhaber: Henkel Kommanditgesellschaft auf Aktien, 40191 Düsseldorf (DE)
(72) Erfinder: HOLLENBERG, Detlef, D-40699 Erkrath (DE); SEIDEL, Kurt, D-40589 Düsseldorf (DE); PRIEBE, Christian, D-42489 Wülfrath (DE)
(86) Internationale Anmeldenummer: EP9600042
(87) Internationale Veröffentlichungsnummer: WO9622072

(56) Entgegenhaltungen:
- EP-A- 0 258 558
- EP-A- 0 692 239
- DE-C- 4 100 490
- DE-C- 4 426 952
- GB-A- 2 206 048

## Beschreibung

Die Erfindung betrifft Mittel, insbesondere kosmetische Mittel, in Form von 2-Phasen-Zubereitungen.

Das menschliche Haupthaar wird heute in vielfältiger Weise mit haarkosmetischen Zubereitungen behandelt. Dazu gehört beispielsweise die Reinigung der Haare mit Shampoos und Duschpräparaten, das Bleichen, Färben und Verformen von Haaren mit Wellmitteln, Tönungsmitteln und Stylingpräparaten sowie die Pflege und Regeneration geschädigter und präventive Behandlung ungeschädigter Haare mit Pflegespülungen und Haarkuren.

Diese Mittel werden in der Mehrzahl der Fälle als Emulsionen oder wäßrige Lösungen formuliert. Emulsionen und wäßrige Lösungen erfüllen in hohem Maße die Erwartungen, die der Verbraucher heutzutage an die sensorischen Eigenschaften und nicht zuletzt auch an das optische Erscheinungsbild von kosmetischen Mitteln, wie z. B. Haarbehandlungsmitteln, stellt. Eine solche Darreichungsform ist aber nur dann möglich, wenn die zu verwendenden Wirkstoffe sowohl mit den verschiedenen Lösungsmitteln als auch untereinander kompatibel sind.

Unter der "Kompatibilität" zweier Stoffe im Sinne der Erfindung ist zu verstehen, daß diese nicht in nennenswertem Umfang in einer Weise reagieren, daß
- einer der beiden oder beide Stoffe merklich chemisch abgebaut werden oder
- beide Stoffe Komplexe bilden, die im vorliegenden Medium schwerlöslich sind und sich daher als Festkörper oder weitere Phase abscheiden.
   Dem Fachmann sind solche Inkompatibilitäten aus der täglichen Praxis bekannt.
   Stoffe sind im Sinne der Erfindung in einer Formulierung insbesondere dann inkompatibel, wenn innerhalb von 12 Stunden nach der Herstellung der Formulierung entweder eine mit bloßem Auge sichtbare Phasenseparierung oder Ausfällung auftritt oder mehr als 50 % des Wirkstoffes abgebaut sind.
   Die Folgen solcher Inkompatibilitäten zeigen sich im Fehlen der durch die Wirkstoffe hervorgerufenen Effekte, zumindest aber in optisch unschönen, den Verbraucher nicht ansprechenden Produkten.
   Es wurde nun überraschenderweise gefunden, daß solche Inkompatibilitäten in vielen Fällen überwunden werden können, wenn die Produkte in Form von speziellen 2-Phasen-Zubereitungen formuliert werden.
   So sind beispielsweise feuchtigkeitsempfindliche Wirkstoffe im Rahmen einer Emulsion applizierbar, wenn diese durch mechanische Bewegung sehr schnell aufgebaut wird und, im Falle mehrfacher Anwendung aus einem Vorratsgefäß, hinreichend schnell wieder bricht. Weiterhin können analog öllösliche und wasserlösliche Wirkstoffe, die in Emulsionen inkompatibel sind, zusammen in einem Mittel appliziert und in Form einer Emulsion auf das Substrat aufgebracht werden.
   Gegenstand der Erfindung ist somit eine in Form eines 2-Phasen-Systems vorliegende, durch mechanische Einwirkung kurzzeitig mischbare Zubereitung zur Behandlung keratinischer Gebilde, insbesondere menschlicher Haare und menschlicher Haut, dadurch gekennzeichnet, daß sie mindestens einen öllöslichen Wirkstoff enthält, der mit einem Bestandteil der wäßrigen Phase inkompatibel ist.
   Das Prinzip kurzzeitig mischbarer 2-Phasen-Formulierungen ist beispielsweise aus den Druckschriften DE-OS 16 17 808, DE-C1-36 27 313, DE-A1-29 05 257, DE-A1-31 10 258, DE-C1-41 00 490 und DE-C1 42 41 799 bekannt. In Mitteln gemäß dieser Druckschriften werden 2-Phasen-Zubereitungen insbesondere deshalb verwendet, um öllösliche Wirkstoffe in Form einer, vom Verbraucher gefühlsmäßig bevorzugten, Emulsion auftragen zu können. Die Herstellung in Form einer stabilen Emulsion ist aber nicht oder nur unter Schwierigkeiten möglich, auf entsprechende Emulgatormengen oder Stabilisierungsmittel soll aus anderen Erwägungen verzichtet werden oder die Emulsionen haben sich bei der Anwendung auf Haut oder Haar als weniger geeignet erwiesen. Die Druckschriften befassen sich aber mit keinem Wort mit Inkompatibilitäten von öllöslichen Wirkstoffen oder deren Feuchtigkeitsempfindlichkeit.
   Im Rahmen der vorliegenden Erfindung ist der Begriff "kurzzeitig mischbar" durch folgenden Test definiert:
   Die 2-Phasen-Zubereitung wird in ein Reagenzglas mit ca. 1 cm Durchmesser in einer solchen Menge gebracht, daß mindestens 90 % des Volumens gefüllt sind. Das verschlossene Reagenzglas wird jeweils durch Drehen um die kurze Achse um 180° 5 mal innerhalb von 5 Sekunden "auf den Kopf gestellt" und wieder in die Ausgangslage gebracht. Dann wurde das Reagenzglas ruhig in der ursprünglichen Lage belassen. Eine 2-Phasen-Zubereitung gilt als "kurzfristig mischbar" im Sinne der Erfindung, wenn bei Betrachtung mit dem bloßen Auge folgende 3 Bedingungen erfüllt wird:
   1. Durch die Bewegung bildet sich ein 1-Phasen-System,
   2. 5 - 30 Sekunden nach Ende der Bewegung ist das Auftreten einer zweiten Phase erkennbar,
   3. nach spätestens 30 Stunden ist die Phasentrennung vollständig abgeschlossen.

Dabei haben sich solche Systeme als anwendungstechnisch besonders geeignet erwiesen, bei denen die Abtrennung der 2. Phase (Bedingung 2) nach 10-20 Sekunden begann und nach 24 Stunden beendet ist (Bedingung 3).

Bei den beiden Phasen kann es sich um eine rein wäßrige Phase, gegebenenfalls mit weiteren gelösten Bestandteilen, und um eine reine Öl-Phase, ebenfalls gegebenenfalls mit weiteren gelösten Bestandteilen handeln.

Üblicherweise wird aber die Wasserphase Ölanteile und die Ölphase Wasseranteile aufweisen.

Dabei ist bevorzugt, daß die wäßrige Phase entweder keine oder nur geringe Ölanteile enthält. Eine solche Emulsion hat dann bevorzugt nicht mehr als 5 Gew.-% Ölanteile, bezogen auf die Komponenten Wasser und Öl.

Als Öl-Phase wird in der Regel eine Emulsion vorliegen, die bis zu 20 Gew.-% Wasser, bezogen auf die Komponenten Öl und Wasser, enthält.

Die erfindungsgemäßen Zubereitungen enthalten die beiden Phasen bevorzugt in einem Verhältnis von 1:1 bis 5:1. Massenverhältnisse von 2:1 bis 1:2 von Öl-Phase und Wasserphase sind dabei besonders bevorzugt.

Öle im Sinne der Erfindung sind mineralische Öle, pflanzliche Öle und synthetische Öle, wie beispielsweise Silikonöle. Wegen ihrer besonderen anwendungstechnischen Eigenschaften kann es bevorzugt sein, als Ölphase Paraffine oder Silikonöle, insbesondere Polydimethylsiloxane niedriger Viskositäten, einzusetzen. Ein entsprechendes Silikonöl ist beispielsweise unter der Bezeichnung Dow Corning^{R}200 Fluid im Handel.

Wirkstoffe im Sinne der Erfindung sind solche Stoffe, die einen kosmetischen Effekt auf der keratinischen Faser, insbesondere dem menschlichen Haar, bewirken. Ausdrücklich keine Wirkstoffe sind Farbstoffe, die lediglich der Anfärbung der Zubereitung dienen, sowie Komponenten, die die Zubereitung vor Zersetzung durch Licht (UV-Filter) oder chemischem Wege (Konservierungsmittel) schützen.

Um die Zubereitungen mit den erfindungsgemäßen Eigenschaften herzustellen, werden diese in der Regel mindestens einen Emulgator enthalten.

Als besonders geeignet für die Herstellung solcher Systeme haben sich beispielsweise kationische derivatisierte Panthenole erwiesen. Solche Verbindungen sind beispielsweise aus der PCT-Offenlegungsschrift WO 92/13829 bekannt, auf die hier ausdrücklich Bezug genommen wird. Ein entsprechendes Produkt wird von der Firma Tri-K unter der Bezeichnung PANTHEQUAT^{R} vertrieben.

Ebenfalls hervorragend geeignet für die Herstellung solcher Systeme sind bestimmte Polyoxyalkylen-Ether von Polyglycerin-Fettsäureestern. Diese Verbindungen enthalten üblicherweise
- 2 bis 20 Oxyalkylen-, insbesondere Oxyethylen-, Einheiten,
- 2 bis 10 Glycerin-Einheiten sowie
- 1 oder 2 Fettsäureeinheiten mit 8 bis 24 Kohlenstoffatomen.

Verbindungen mit bis zu 5 Glycerin-Einheiten, Oxyethylen-Einheiten und einer Fettsäureeinheit haben sich als erfindungsgemäß besonders geeignet erwiesen. Die Fettsäureeinheiten können sowohl gesättigt als auch ungesättigt sein; Verbindungen auf Basis Laurin-, Myristin-, Palmitin-, Stearin- und Öl-Säure haben sich als besonders geeignet erwiesen.

Emulgatoren dieses Typs sind ebenfalls im Handel erhältlich; so vertreibt die Hoechst AG unter der Bezeichnung Hostacerin^{R}-DGL einen Poly(10)ethylenglykolether eines mit Laurinsäure veresterten Diglycerins.

Diese Emulgatoren sind in den erfindungsgemäßen Zubereitungen bevorzugt in einer Menge von 0,05 bis 1 Gew.-%, insbesondere 0,05 bis 0,5 Gew-%, bezogen auf die gesamte Zubereitung, enthalten.

Dem Fachmann sind eine Reihe von Wirkstoffklassen bekannt, bei denen öllösliche Vertreter mit Wasser oder wasserlöslichen Verbindungen inkompatibel sind.

Beispiele für solche Wirkstoffklassen sind beispielsweise Ester, Vitamine, Parfumöle, kationische Tenside und Proteinderivate.

Eine Reihe von Estern, beispielsweise bestimmte Vitamin A- und Vitamin E-Ester, sind in kosmetischen Emulsionen nicht hinreichend hydrolysebeständig, um eine solche Formulierung zu erlauben.

Weiterhin sind bestimmte Parfümkomponenten, insbesondere solche mit Keto- oder Aldehydgruppen, inkompatibel mit bestimmten wasserlöslichen Wirkstoffen. Ein solcher Wirkstoff ist beispielsweise das häufig als Antischuppenmittel verwendete Piroctone Olamine, das unter dem Warenzeichen Octopirox^{R} vermarktet wird.

Unverträglichkeiten treten ebenfalls auf bei Zugabe bestimmter kationischer Tenside zu Formulierungen mit anionischen Tensiden. Beispiele für solche kationischen Tenside sind quartäre Ammoniumverbindungen mit 3 langen Kohlenstoffketten, wie Tricetylmethylammoniumchlorid.

Als unverträglich mit kationischen Tensiden vom Typ der Cetyltrimethylammoniumchloriden dagegen haben sich bestimmte öllösliche Protein-Derivate wie z. B. Kondensate von Kollagenhydrolysaten mit Ölsäure erwiesen. Solche Substanzen sind beispielsweise unter der Bezeichnung Lamepon^{R} LPO im Handel.

Zubereitungen, die als öllöslichen Wirkstoff einen Vertreter der vorgenannten Klassen enthalten, sind im Rahmen der Erfindung bevorzugt.

Gegenstand einer bevorzugten Ausführungsform der Erfindung sind solche Zubereitungen, bei denen der öllösliche Wirkstoff mit Wasser selbst inkompatibel ist.

Gegenstand einer weiteren bevorzugten Ausführungsform der Erfindung sind solche Zubereitungen, bei denen der öllösliche Wirkstoff mit einem wasserlöslichen Wirkstoff inkompatibel ist, ausgewählt aus der Gruppe, die von anionischen Tensiden, kationischen Tensiden und Antischuppenmitteln gebildet wird.

Die Vorteile der erfindungsgemäßen Zubereitungen lassen sich eindrucksvoll anhand folgender Wirkstoffkombinationen belegen:

Vitamin-A-Derivate, z. B. Vitamin-A-palmitat (öllöslicher Wirkstoff), verbessern die natürlichen Hautfunktionen, erhöhen die Hautelastizität und normalisieren den Keratinisierungsprozeß; Piroctone Olamine (wasserlöslicher Wirkstoff) ist aufgrund seiner antibakteriellen Eigenschaften ein hervorragendes Antischuppenmittel. Durch Kombination beider, miteinander unverträglicher Stoffe wird die pilzlich bedingte Kopfhautschuppung in synergistischer Weise reduziert; eine Formulierung als 2-Phasen-Zubereitung ermöglicht die gleichzeitige Applikation beider Wirkstoffe aus einem einzigen, lagerstabilen Mittel.

Quartäre Ammoniumverbindungen vom Typ der Tricetylmethylammoniumchloride verringern als Wirkstoffe in hervorragender Weise die statische Aufladung des Haars, Aniontenside vom Typ der Alkylsulfate und Alkylethersulfate bewirken eine gute Reinigung des Haares, kationische Polymere vom Typ der kationischen Celluloseether (Marktprodukt: Polymer JR^{R}) haben eine ausgezeichnete Avivagewirkung. 2-Phasen-Zubereitungen, die in der Ölphase den erstgenannten Wirkstoff, in der wäßrigen Phase die beiden anderen Wirkstoffe enthalten, eignen sich in herausragender Weise für die gleichzeitige Reinigung und Konditionierung von Haaren.

Die öllöslichen Wirkstoffe sind in den erfindungsgemäßen Zubereitungen in den dem Fachmann bekannten, für die gewünschte Wirkung erforderlichen Mengen enthalten. Bevorzugte Einsatzmengen liegen bei 0,05 bis 5 Gew.-%, insbesondere bei 0,05 bis 2 Gew.-%, bezogen auf die gesamte Zubereitung.

Entsprechend der Aufgabe der erfindungsgemäßen Zubereitung enthält diese in der Regel noch weitere kosmetische Wirkstoffe, sowie übliche Hilfs- und Zusatzstoffe. In den meisten Fällen unterliegen die erfindungsgemäßen Zubereitungen hinsichtlich dieser Bestandteile keinen Beschränkungen. Lediglich bei weiteren Tensiden, die das Mischungsverhalten der beiden Phasen und die Emulsionsstabilität stark beeinflussen können, muß der Fachmann die Eignung mit Hilfe des oben genannten einfachen Testes gegebenenfalls überprüfen; gleiches gilt für den Fall, daß große Mengen an viskositätssteigernden Mitteln eingesetzt werden sollen.

Übliche Wirk-, Hilfs- und Zusatzstoffe für die erfindungsgemäßen Zubereitungen sind beispielsweise
- anionische Tenside, wie beispielsweise Fettalkylsulfate- und -ethersulfate sowie Alkylethercarbonsäuren,
- zwitterionische Tenside, wie beispielsweise Betaine,
- ampholytische Tenside,
- nichtionogene Tenside, wie beispielsweise Alkylpolyglycoside und ethoxylierte Fettalkohole,
- kationische Tenside, wie beispielsweise quartäre Ammoniumverbindungen, Amidoamine und sogenannte "Esterquats"
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecithin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Zink Omadine,
- weitere Substanzen zur Einstellung des pH-Wertes,
- Wirkstoffe wie Panthenol, Allantoin, Pyrrolidoncarbonsäuren und deren Salze und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Die erfindungsgemäßen Zubereitungen unterliegen hinsichtlich der Art des Mittels keinerlei weiteren Beschränkungen. Bevorzugt sind aber kosmetische Mittel, insbesondere Haarbehandlungsmittel. Innerhalb der Gruppe der Haarbehandlungsmittel sind die erfinderischen Zubereitungen insbesondere geeignet für Haarpflegeprodukte wie Haarkuren, Haarbalsame etc., die ohne weiteres Spülen auf dem Haar verbleiben. Gegenstand der Erfindung ist daher ebenfalls ein Verfahren zur Behandlung von Haaren, bei dem eine erfindungsgemäße Zubereitung auf das Haar aufgebracht wird und dort verbleibt.

Es können aber auch Produkte formuliert werden, die nach einer gewissen Einwirkzeit (üblicherweise ca. 30 Sekunden bis ca. 30 Minuten) wieder aus dem Haar ausgespült werden. Gegenstand der Erfindung ist daher auch ein Verfahren zur Behandlung von Haaren, bei dem eine erfindungsgemäße Zubereitung auf das Haar aufgebracht und nach einer Einwirkzeit wieder ausgespült wird.

Die folgenden Beispiele sollen die Erfindung näher erläutern.

### Beispiele

### Formulierungsbeispiele:

Alle Angaben sind in Gewichts-%.

### 1. Haarkur:

| Ölphase: | |
|---|---|
| Dow Corning^{R}345¹ | 24,5 |
| Parfumöl (keton- und aldehydhaltig | 0,5 |

| Wasserphase: | |
|---|---|
| Wasser | 64,72 |
| Ethanol | 8,0 |
| Propylenglykol | 1,0 |
| Hostacer in^{R}DGS² | 0,4 |
| Merquat^{R}550³ | 0,8 |
| Octopirox^{R} ⁴ | 0,08 |

| | |
|---|---|
| ¹ Decamethylpentasiloxan (CTFA-Bezeichnung: Cyclomethicone) (DOW CORNING) | |
| ² Fettsäurepolyglycerinester-EO-Addukt (CTFA-Bezeichnung: PEG-4 polyglyceryl-2 Stearate) (HOECHST) | |
| ³ Dimethyldiallylammoniumchlorid-Acrylamid-Copolymer (ca. 8,5 % Aktivsubstanz in Wasser; CTFA-Bezeichnung: Polyquaternium-7) (MERCK) | |
| ⁴ Hydroxy-4-methyl-6(2,4,4-trimethylpentyl)-2-pyridon-Monoethanolamin-Salz (CTFA-Bezeichnung: Piroctone Olamine) (HOECHST) | |

### 2. Haarkur:

| Ölphase: | |
|---|---|
| Dow Corning^{R}200⁵ 0,65cSt. | 32,65 |
| Cetiol^{R}0E⁶ | 2,0 |
| Vitamin E-acetat | 0,3 |
| Vitamin A palmitat | 0,05 |

| Wasserphase: | |
|---|---|
| Wasser | 63,7 |
| Pathequat^{R 7} | 0,2 |
| Phenoxyethanol | 0,8 |
| Panthenol | 0,2 |
| Celquat^{R}L 200⁸ | 0,1 |

| | |
|---|---|
| ⁵ Hexamethyldisiloxane (CTFA-Bezeichnung: Dimethicone) (DOW CORNING) | |
| ⁶ Dioctylether (CTFA-Bezeichnung: Dicaprylether) (HENKEL) | |
| ⁷ kationisches Panthenolderivat (45 % Aktivsubstanz; CTFA-Bezeichnung: Panthenyl Hydroxypropylsteardimoniumchlorid) (TRI-K Industries) | |
| ⁸ Hydroxyethylcellulose-Diallyldimethylammoniumchlorid-Copolymere (95 % Aktivsubstanz; CTFA-Bezeichnung: Polyquaternium-4) (DELFT-NATIONAL) | |

## Patentansprüche

1. In Form eines 2-Phasen-Systems vorliegende Zubereitung zur Behandlung keratinischer Gebilde, insbesondere menschlichen Haaren und menschlicher Haut, dadurch gekennzeichnet,
- daß sie mindestens einen öllöslichen Wirkstoff enthält, der mit einem Bestandteil der wäßrigen Phase in der Form inkompatibel ist, daß einer oder beide Stoffe merklich chemisch abgebaut werden oder beide Stoffe Komplexe bilden, die im vorliegenden Medium schwerlöslich sind und sich als Festkörper oder weitere Phase abscheiden, und
- daß das 2-Phasen-System durch mechanische Einwirkung kurzzeitig mischbar ist unter der Bedingung, daß 5 - 30 Sekunden nach Ende der mechanischen Einwirkung das Auftreten einer zweiten Phase erkennbar ist.

2. Zubereitung nach Anspruch 1, dadurch gekennzeichnet, daß eine Phase Wasser oder eine Emulsion ist, die bis zu 5 Gew.-% Öl, bezogen auf die Komponenten Wasser und Öl, enthält.

3. Zubereitung nach Anspruch 1 oder 2, dadurch gekennzeichnet, daß die Öl-Phase bis zu 20 Gew.-% Wasser, bezogen auf die Komponenten Öl und Wasser, enthält.

4. Zubereitung nach einem der Ansprüche 1 bis 3, dadurch gekennzeichnet, daß die beiden Phasen in einem Massen-Verhältnis von 1:1 bis 5:1 vorliegen.

5. Zubereitung nach einem der Ansprüche 1 bis 4, dadurch gekennzeichnet, daß der öllösliche Wirkstoff ausgewählt ist aus der Gruppe, die Vitamine, Parfumöle, kationische Tenside und Proteinderivate umfaßt.

6. Zubereitung nach einem der Ansprüche 1 bis 5, dadurch gekennzeichnet, daß der öllösliche Wirkstoff mit Wasser inkompatibel ist.

7. Zubereitung nach einem der Ansprüche 1 bis 5, dadurch gekennzeichnet, daß der inkompatible Bestandteil der wäßrigen Phase ausgewählt ist aus der Gruppe, die von anionischen Tensiden, kationischen Tensiden und Antischuppenmitteln gebildet wird.

8. Zubereitung nach einem der Ansprüche 1 bis 7, dadurch gekennzeichnet, daß der öllösliche Wirkstoff in Mengen von 0,05 bis 5 Gew.-%, insbesondere 0,05 bis 2 Gew.-%, bezogen auf die gesamte Zubereitung, enthalten ist.

9. Verfahren zur Behandlung von Haaren, dadurch gekennzeichnet, daß eine Zubereitung nach einem der Ansprüche 1 bis 8 auf das Haar aufgebracht wird und dort verbleibt.

10. Verfahren zur Behandlung von Haaren, dadurch gekennzeichnet, daß eine Zubereitung nach einem der Ansprüche 1 bis 8 auf das Haar aufgebracht wird und nach einer Einwirkzeit wieder ausgespült wird.

## Revendications

1. Préparation se présentant sous la forme d'un système en 2 phases en vue du traitement des structures kératiniques, en particulier des cheveux humains et de la peau humaine,
caractérisée en ce qu'
- elle contient au moins un principe actif soluble dans l'huile qui est incompatible avec un constituant de la phase aqueuse, du fait qu'une ou les deux substances sont dégradées notablement par voie chimique ou que les deux substances forment des complexes qui sont peu solubles dans le présent milieu et qui se séparent sous forme de composés solides ou d'une autre phase, et
- le système à 2 phases est miscible par action mécanique de courte durée dans les conditions qui font que 5 à 30 secondes après la fin de l'action mécanique, la survenue d'une deuxième phase peut être décelée.

2. Préparation selon la revendication 1,
caractérisée en ce qu'
une phase est de l'eau ou une émulsion qui renferme jusqu'à 5 % en poids d'huile rapporté aux composants eau et huile.

3. Préparation selon l'une quelconque des revendications 1 ou 2,
caractérisée en ce que
la phase huileuse renferme jusqu'à 20 % en poids d'eau, rapporté aux composants huile et eau.

4. Préparation selon l'une quelconque des revendications 1 à 3,
caractérisée en ce que
les deux phases sont présentes dans un rapport en masse allant de 1 : 1 à 5 : 1.

5. Préparation selon l'une quelconque des revendications 1 à 4,
caractérisée en ce que
le principe actif soluble dans l'huile est choisi dans le groupe qui comprend les vitarnines, les essences de parfum, les agents tensioactifs cationiques et les dérivés de protéine.

6. Préparation selon l'une quelconque des revendications 1 à 5,
caractérisée en ce que
le principe actif soluble dans l'huile est incompatible avec l'eau.

7. Préparation selon l'une quelconque des revendications 1 à 5,
caractérisée en ce que
le constituant incompatible de la phase aqueuse est choisi dans le groupe qui est formé des agents tensloactifs anioniques, des agents tensioactifs cationiques et des agents antipelliculaires.

8. Préparation selon l'une quelconque des revendications 1 à 7,
caractérisée en ce que
le principe actif soluble dans l'huile est contenu en quantités allant de 0,05 à 5 % en poids, en particulier de 0,05 à 2 % en poids rapporté à la préparation totale.

9. Procédé de traitement des cheveux,
caractérisé en ce qu'
on applique une préparation selon l'une des revendications 1 à 8 sur les cheveux et elle y demeure.

10. Procédé de traitement des cheveux,
caractérisé en ce qu'
on applique une préparation selon l'une des revendications 1 à 8 sur les cheveux, et on l'élimine par rinçage à nouveau après un temps d'action.

## Claims

1. A preparation in the form of a two-phase system for the treatment of keratin structures, more particularly human hair and human skin, characterized in that it contains
- at least one oil-soluble active principle which is incompatible with a constituent of the aqueous phase in the sense that one or both substances are chemically degraded to a significant extent or both substances form complexes which are poorly soluble in the present medium and separate as solids or as another phase, and
- the two-phase system can be mixed in a short time by mechanical action on the condition that the appearance of a second phase is discernible 5 to 30 seconds after the end of the mechanical action.

2. A preparation as claimed in claim 1, characterized in that one phase is water or an emulsion which contains up to 5% by weight of oil, based on the components water and oil.

3. A preparation as claimed in claim 1 or 2, characterized in that one phase is an oil phase which contains up to 20% by weight of water, based on the components oil and water.

4. A preparation as claimed in any of claims 1 to 3, characterized in that the two phases are present in a ratio by weight of 1:1 to 5:1.

5. A preparation as claimed in any of claims 1 to 4, characterized in that the oil-soluble active principle is selected from the group consisting of vitamins, perfume oils, cationic surfactants and protein derivatives.

6. A preparation as claimed in any of claims 1 to 5, characterized in that the oil-soluble active principle is incompatible with water.

7. A preparation as claimed in any of claims 1 to 5, characterized in that the incompatible constituent of the aqueous phase is selected from the group consisting of anionic surfactants, cationic surfactants and antidandruff agents.

8. A preparation as claimed in any of claims 1 to 7, characterized in that the oil-soluble active principle is present in quantities of 0.05 to 5% by weight and, more particularly, 0.05 to 2% by weight, based on the preparation as a whole.

9. A hair treatment process, characterized in that the preparation claimed in any of claims 1 to 8 is applied to and left on the hair.

10. A hair treatment process, characterized in that the preparation claimed in any of claims 1 to 8 is applied to the hair and, after a contact time, is rinsed off.
